Europäisches Patentamt

European Patent Office

Office européen des brevets

(19)

(11) Numéro de publication : **0 109 903**
**B1**

(12) **FASCICULE DE BREVET EUROPÉEN**

(45) Date de publication du fascicule du brevet :
19.08.87

(21) Numéro de dépôt : 83402211.3

(22) Date de dépôt : 17.11.83

(51) Int. Cl.⁴ : **A 61 M 5/14**

(54) **Raccords pour injections extemporanées.**

(30) Priorité : 19.11.82 FR 8219445

(43) Date de publication de la demande :
30.05.84 Bulletin 84/22

(45) Mention de la délivrance du brevet :
19.08.87 Bulletin 87/34

(84) Etats contractants désignés :
AT BE CH DE FR GB IT LI LU

(56) Documents cités :
FR-A- 2 345 141
FR-A- 2 425 248
GB-A- 2 085 732
US-A- 4 000 740
US-A- 4 048 996

(73) Titulaire : **LABORATOIRES BRUNEAU Société anonyme dite:**
**204 avenue du Maréchal Juin**
**F-92100 Boulogne Billancourt (FR)**

(72) Inventeur : **Leclerc, Roland**
**"La Groie"**
**F-28400 Nogent le Rotrou (FR)**

(74) Mandataire : **Combe, André et al**
**CABINET BEAU DE LOMENIE 55, rue d'Amsterdam**
**F-75008 Paris (FR)**

Jouve, 18, rue St-Denis, 75001 Paris, France

## Description

La présente invention concerne un nouveau raccord utilisable pour les injections extemporanées.

Durant l'opération de perfusion ou de transfusion à laquelle les malades sont fréquemment soumis, il est souvent souhaitable d'injecter, de façon discontinue ou continue, dans le liquide de perfusion ou de transfusion, des quantités données de liquides divers, par exemple des médicaments.

Cette injection s'effectue généralement au moyen d'une seringue et d'une aiguille ou d'un système annexe de perfusion. Divers dispositifs existent sur le marché, faisant appel pour l'accès à la voie de perfusion ou de transfusion soit à une dérivation fermée en temps normal par un bouchon ou un robinet, soit à un raccord intercalé dans la ligne et comportant au moins une partie s'auto-étanchant après perforation par une aiguille et retrait de celle-ci.

Ces systèmes présentent souvent un certain nombre de défauts dont, par exemple, l'existence d'un volume interne important, cause d'un risque de rétention puis de libération de bulles d'air dans le liquide administré, ou la possibilité d'injecter le produit à contre-courant du sens normal du liquide (US-A-4 048 996) ou le trop faible parcours libre de l'aiguille avec le risque de détérioration de sa pointe contre une paroi dure, ou de sa pénétration dans l'épaisseur d'une paroi souple, ou la nécessité, lors d'une injection extemporanée, pour éviter le reflux, vers l'amont, du liquide ainsi injecté, de fermer le régulateur de débit (ce qui implique un nouveau réglage ultérieur dudit régulateur) ou de placer, entre le régulateur et le raccord un système annexe de fermeture ou encore de juxtaposer ou d'incorporer au raccord un dispositif relativement complexe de valve antiretour (GB-A-2 085 732).

Le raccord selon la présente invention permet de remédier à ces divers inconvénients.

Ledit raccord est formé par l'assemblage étanche et définitif de deux pièces en matière plastique, de préférence transparente ou translucide, la première pièce étant constituée par un tube destiné à recevoir un tuyau souple plastique d'amenée du liquide de perfusion ou transfusion, et par un plateau perpendiculaire audit tube ; la deuxième pièce étant constituée par un tube destiné à recevoir un tuyau plastique de départ du liquide de perfusion ou transfusion, et par un plateau perpendiculaire au tube de la deuxième pièce et comportant une saignée sur sa face interne assurant le passage du liquide arrivant du tube de la première pièce vers le tube de la deuxième pièce, le plateau de la première pièce comportant, dans une position située sensiblement dans l'axe du tube de la deuxième pièce lorsque les deux pièces sont assemblées, un logement troué permettant l'insertion d'un opercule autoobturable permettant l'injection de substances étrangères, l'assemblage des deux pièces étant réalisé de façon définitive et étanche en assurant également le maintien étanche dudit opercule auto-obturable dans ledit logement, ledit raccord étant caractérisé en ce que

ledit plateau de la deuxième pièce comporte, dans une position située sensiblement à l'opposé du tube de la première pièce par rapport à l'ensemble constitué par les deux plateaux lorsque les deux pièces sont assemblées, un dispositif fixe comportant une fente et permettant la fermeture, par pincement, du tuyau souple d'amenée du liquide de perfusion ou transfusion, ledit tuyau souple étant coincé dans ladite fente,

la disposition de l'opercule auto-obturable par rapport au tube de départ du liquide n'autorise l'injection extemporanée que dans le sens de l'accès veineux du patient.

Le plateau de la deuxième pièce porte sur sa face externe (c'est-à-dire la face du plateau qui n'est pas assemblée au plateau de la première pièce), un dispositif de pincement du tuyau souple amont raccordé au dispositif selon l'invention.

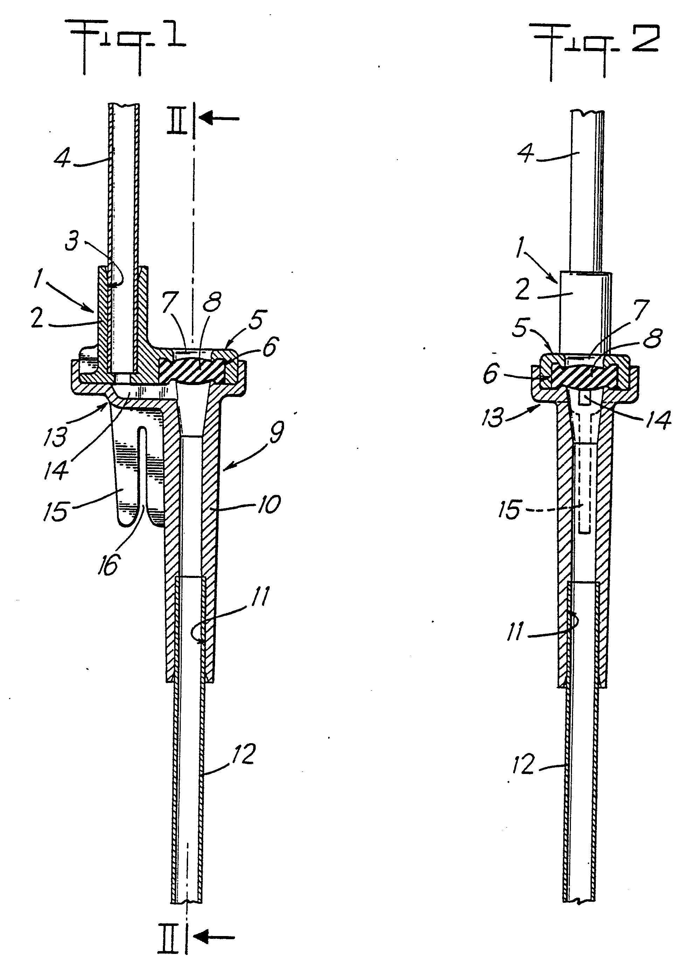

L'invention sera mieux comprise en se référant aux figures 1 et 2 qui représentent un mode de réalisation de l'invention sous forme de raccord terminé (les deux plateaux des pièces ayant été assemblés définitivement entre eux) ;

la figure 1 est une élévation en coupe du raccord ;

la figure 2 est une vue de ce raccord, en coupe, selon II-II.

Sur les figures 1 et 2, on voit :

en 1 la première pièce comportant un tube 2 muni d'un chambrage 3 dans lequel vient s'adapter le tuyau plastique amont 4 du perfuseur ou transfuseur,

en 5 le plateau de ladite première pièce qui s'étend perpendiculairement au tube 2 ; dans ce plateau, on a aménagé, au côté du tube 2, une chambre 6 comportant une ouverture 7, chambre dans laquelle on admet l'opercule en matière auto-obturable 8 ; on entend par matière auto-obturable toute substance qui se referme après perforation par une aiguille de dimension habituellement utilisée et retrait de celle-ci,

en 9 la deuxième pièce comportant un tube 10 muni d'un chambrage 11 dans lequel vient s'adapter le tuyau plastique aval 12 du perfuseur ou transfuseur ; le chambrage 11 est surmonté d'une tubulure en forme cylindrique ou très légèrement tronconique qui se trouve, lorsque les deux pièces sont assemblées de façon définitive, directement au-dessous du caoutchouc 8 ;

en 13 le plateau de la seconde pièce qui s'étend perpendiculairement au tube 10 ; ce plateau 13 comporte, sur sa face interne (celle qui sera en regard, après montage, de la face correspondante de la pièce 1) une saignée 14 ;

sur la face extérieure de cette deuxième pièce 9, on a disposé une excroissance 15 en matière plastique également comportant une fente 16 dans laquelle on peut venir coincer pour l'obturer le tuyau 4 lorsque l'on réalise l'injection, par l'opercule 8, d'un médicament quelconque dans le tube 10 de la pièce 9.

Les deux pièces 1 et 9 sont assemblées définitivement l'une à l'autre (par exemple par soudure ultrason), de façon que les tubes 2 et 10 soient parallèles et que l'opercule 8 soit maintenu en place dans la chambre 6 du plateau de la pièce 1. Dans ces conditions, la saignée 14 du plateau 13 forme, avec la face en regard du plateau 5, un canal qui relie les extrémités ouvertes des tubes 2 et 10.

Le fonctionnement du raccord ainsi réalisé est très simple. Le tuyau souple amenant le liquide de perfusion ou transfusion est branché dans le tube 2 et le tuyau souple allant au patient est branché dans le tube 10. La perfusion ou transfusion est réalisée normalement, c'est-à-dire que le réglage du débit du liquide est effectué à l'aide d'un dispositif connu (par exemple une pince) disposé sur le tuyau souple amenant le liquide de perfusion ou transfusion. Lorsque l'on veut injecter un produit dans ledit liquide, on procède aux opérations suivantes : on coince le tuyau d'amenée du liquide de perfusion ou transfusion dans la fente 16 de l'excroissance 15 de façon à stopper l'arrivée dudit liquide, si cela est jugé nécessaire.

On introduit le produit à injecter à l'aide d'une seringue ou d'un système annexe de perfusion muni(e) d'une aiguille que l'on pique à travers l'opercule 8 ; on remarquera que le fait, pour le raccord selon l'invention, d'être en matériau translucide ou transparent permet de voir généralement la position de la pointe de l'aiguille à l'intérieur du raccord et que, de toute façon, la deuxième pièce 9 est prévue (existence d'une tubulure de forme cylindrique ou tronconique directement au-dessous de l'opercule 8 de façon qu'il n'y ait aucun risque de détérioration ni de la pointe de l'aiguille ni du tuyau souple aval par ladite pointe de l'aiguille ;

puis on enlève le tuyau d'amenée du liquide de perfusion ou transfusion de la fente 16 dans laquelle il a été éventuellement initialement coincé ; dans ces conditions, le liquide de perfusion ou transfusion recommence à circuler avec un débit sensiblement égal au débit qui avait été préalablement réglé à l'aide d'un dispositif connu.

**Revendication**

Raccord pour injections extemporanées formé par l'assemblage de deux pièces (1, 9) en matière plastique, de préférence translucide ou transparente, la première pièce (1) étant constituée par un tube (2) destiné à recevoir un tuyau souple plastique (4) d'amenée du liquide de perfusion ou transfusion, et par un plateau (5) perpendiculaire audit tube (2) ; la deuxième pièce (9) étant constituée par un tube (10) destiné à recevoir un tuyau (12) plastique de départ du liquide de perfusion ou transfusion, et par un plateau (13) perpendiculaire au tube (10) de la deuxième pièce (9) et comportant une saignée (14) sur sa face interne assurant le passage du liquide arrivant du tube (2) de la première pièce (1) vers le tube (10) de la deuxième pièce (9), le plateau (5) de la première pièce (1) comportant, dans une position située sensiblement dans l'axe du tube (10) de la deuxième pièce (9) lorsque les deux pièces (1, 9) sont assemblées, un logement (6) troué (7) permettant l'insertion d'un opercule auto-obturable (8) permettant l'injection de substances étrangères, l'assemblage des deux pièces (1, 9) étant réalisé de façon définitive et étanche en assurant également le maintien étanche dudit opercule auto-obturable (8) dans ledit logement (6), ledit raccord étant caractérisé en ce que

ledit plateau (13) de la deuxième pièce (9) comporte, dans une position située sensiblement à l'opposé du tube (2) de la première pièce (1) par rapport à l'ensemble constitué par les deux plateaux (5, 13) lorsque les deux pièces (1, 9) sont assemblées, un dispositif fixe (15, 16) comportant une fente (16) et permettant la fermeture, par pincement, du tuyau souple (4) d'amenée du liquide de perfusion ou transfusion, ledit tuyau souple (4) étant coincé dans ladite fente (16),

la disposition de l'opercule auto-obturable (8) par rapport au tube (10) de départ du liquide n'autorise l'injection extemporanée que dans le sens de l'accès veineux du patient.

**Claim**

A connecting element for extemporaneous injections constituted by the assembly of two pieces (1, 9) in plastic material, preferably translucid or transparent, the first piece (1) being constituted by a tube (2) adapted to receive a supple plastic pipe (4) supplying perfusion or transfusion liquid, and by a plate (5) perpendicular to said tube (2) ; the second piece (9) being constituted by a tube (10) designed to receive a plastic tube (12) wherefrom the perfusion or transfusion liquid flows, and by a plate (13) perpendicular to the tube (10) of the second piece (9) and comprising a hole (14) on its inner face through which flows the liquid issuing from the tube (2) of the first piece (1) towards the tube (10) of the second piece (9), the plate (5) of the first piece (1) comprising, in a position situated substantially along the axis of the tube (10) of the second piece (9) when the two pieces (1, 9) are assembled together, a perforated (7) housing (6) permitting the insertion of a selfclosable cap (8) for injecting extraneous substances, assembly of the two pieces (1, 9) being achieved in permanent and tight manner, while also providing for a tight holding of said selfclosable cap (8) inside said housing (6), said connecting element being characterized in that said plate (13) of the second piece (9) comprises, in a position situated substantially opposite the tube (2) of the first piece (1) with respect to the assembly constituted by the two plates (5, 13) when said two pieces (1, 9) are assembled together, a fixed device (15, 16) comprising a slot (16) permitting the closure, by squeezing, of the supple tube (4) supplying the perfusion or transfusion liquid, said supple tube (4) being wedged in said slot (16),

the disposition of the self-closable cap (8) with respect to the liquid-supplying tube (10) allows for the extemporaneous injection only in the direction of the access to the venous system of the patient.

## Patentanspruch

Anschlußstück zum Einspritzen zusätzlicher Medien, in Form zweier zusamnengefügter Stücke (1, 9) aus vorzugsweise durchscheinendem oder transparentem Plastikmaterial, wobei das erste Stück (1) durch ein Rohr (2), das zur Aufnahme eines Plastikschlauchs (4) zur Zuführung von Perfusions- oder Transfusionsflüssigkeit bestimmt ist, und durch eine zum Rohr (2) senkrecht stehende Platte (5) gebildet ist ; wobei das zweite Stück (9) durch ein Rohr (10), das zur Aufnahme eines Plastikschlauchs (12) zur Abgabe der Perfusions- oder Transfusionsflüssigkeit bestimmt ist, und durch eine zum Rohr (10) des zweiten Stücks (9) senkrecht stehende Platte (13) gebildet ist, die an ihrer Innenseite einen Einschnitt (14) zur Gewährleistung des Durchtritts der vom Rohr (2) des ersten Stücks (1) kommenden Flüssigkeit zum Rohr (10) des zweiten Stücks (9) hin aufweist, wobei die Platte (5) des ersten Stücks (1) in einer im wesentlichen in der Achse des Rohrs (10) des zweiten Stücks (9) befindlichen Position, wenn die beiden Stücke (1, 9) zusammengefügt sind, einen Sitz (6) mit Loch (7) zum Einsetzen eines selbstschließenden Innendeckels (8) aufweist, der die Injizierung von Fremdsubstanzen ermöglicht, wobei die beiden Stücke (1, 9) auf endgültige und dichte Weise zusammengefügt sind und gleichzeitig die dichte Halterung des selbstschließenden Innendeckels (8) im Sitz (6) gesichert ist, welches Anschlußstück dadurch gekennzeichnet ist, daß

die Platte (13) des zweiten Stücks (9) in einer in bezug auf die Gesamtheit aus beiden Platten (5, 13), wenn die beiden Stücke (1, 9) zusammengefügt sind, im wesentlichen vom Rohr (2) des ersten Stücks (1) abgewandten Stellung eine feststehende Anordnung (15, 16) umfaßt, die einen Schlitz (16) aufweist und das Verschließen des Schlauchs (4) zur Zuführung von Perfusions- oder Transfusionsflüssigkeit durch Klemmen ermöglicht, wobei der Schlauch (4) im Schlitz (16) eingeklemmt ist,

die Anordnung des selbstschließenden Innendeckels (8) gegenüber dem Rohr (10) zur Abgabe der Flüssigkeit die zusätzliche Injizierung nur im Sinne eines Eintritts in die Vene des Patienten gestattet.

# Fig.1

# Fig.2